(19) Europäisches Patentamt / European Patent Office / Office européen des brevets

(11) **EP 3 218 036 B1**

(12) **FASCICULE DE BREVET EUROPEEN**

(45) Date de publication et mention
de la délivrance du brevet:
**05.12.2018 Bulletin 2018/49**

(21) Numéro de dépôt: **16729007.1**

(22) Date de dépôt: **28.04.2016**

(51) Int Cl.:
*A61M 16/00* *(2006.01)*    *A61H 31/00* *(2006.01)*

(86) Numéro de dépôt international:
**PCT/FR2016/051000**

(87) Numéro de publication internationale:
**WO 2016/198757 (15.12.2016 Gazette 2016/50)**

(54) **APPAREIL DE VENTILATION ARTIFICIELLE AVEC MONITORAGE D'UNE ABSENCE DE CONTRACTIONS THORACIQUES**

VORRICHTUNG ZUR KÜNSTLICHEN BEATMUNG MIT ÜBERWACHUNG AUF ABWESENHEIT VON BRUSTKONTRAKTIONEN

APPARATUS FOR ARTIFICIAL VENTILATION, WITH MONITORING FOR ABSENCE OF CHEST CONTRACTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.06.2015 FR 1555191**

(43) Date de publication de la demande:
**20.09.2017 Bulletin 2017/38**

(73) Titulaire: **Air Liquide Medical Systems**
**92160 Antony (FR)**

(72) Inventeurs:
• **JACQUOT, Eric**
**92160 Antony (FR)**
• **PENNORS, Thomas**
**92160 Antony (FR)**
• **RIGOLLOT, Marceau**
**92160 Antony (FR)**
• **RICHARD, Jean-Christophe**
**92160 Antony (FR)**

(74) Mandataire: **Pittis, Olivier**
**L'Air Liquide, S.A.**
**Direction de la Propriété Intellectuelle**
**75, Quai d'Orsay**
**75321 Paris Cedex 07 (FR)**

(56) Documents cités:
EP-A1- 2 198 823    EP-A2- 2 343 097
WO-A1-00/20061    WO-A1-2013/025406
WO-A1-2014/095962    FR-A1- 3 000 893
US-A1- 2013 310 718

**Description**

L'invention concerne un appareil de ventilation artificielle utilisable lors d'un massage cardiaque sur un patient ventilé, pour assurer un monitorage, c'est-à-dire un suivi, du massage cardiaque pratiqué par un secouriste, tel un médecin du SAMU, un pompier, un infirmier ou analogue, en particulier un monitorage du temps sans compression. Les documents FR 3 000 893 A1, WO 2013/025406 A1, US 2013/310718 A1 et EP 2 198 823 A1 décrivent différents dispositifs connus de l'art antérieur qui sont utilisables par des secouristes lors d'un massage cardiaque sur un patient.

En cas d'arrêt cardiaque, lorsqu'une réanimation cardio-pulmonaire (RCP) est entreprise, les secouristes doivent s'attacher à interrompre le moins possible les compressions thoraciques dans le but de ne jamais stopper le débit cardiaque généré par les compressions thoraciques et de ne pas désamorcer la pompe cardiaque.

Le temps dit de « no-flow » ou « sans-flux », c'est-à-dire le temps pendant lequel le patient est sans débit cardiaque, est un indicateur de pronostic fort pour la survie sans séquelle du patient.

Il faut distinguer le « no-flow » initial (ou $tNF_{init}$) correspondant au temps (i.e. durée) écoulé entre la survenue de l'arrêt cardiaque et les premières compressions thoraciques réalisées, du « no flow » total (ou $tNF_{tot}$) qui comprend aussi toutes les interruptions de compressions thoraciques (CT) pendant la prise en charge par les secours professionnels, c'est-à-dire un secouriste, comme un pompier, un médecin du SAMU ou autre, encore appelée « RCP spécialisée ». Chaque temps individuel d'interruption des CT est appelé « temps sans CT » ou « temps no-CT » (ou tNCT).

Ceci se traduit alors par l'équation suivante :
$$tNF_{tot} = tNF_{init} + S(tNCT)$$
où : S(tNCT) est la somme de tous les temps d'interruption des CT.

Sachant, en outre, qu'une phase de RCP spécialisée nécessite, systématiquement la mise en oeuvre d'une ventilation artificielle réalisée dans la plupart des cas par un appareil d'assistance respiratoire délivrant un gaz respiratoire, tel de l'air, au patient via une interface patient, tel un masque ou une sonde d'intubation, les occasions d'interrompre le massage cardiaque sont donc nombreuses, notamment intubation, analyse de rythme du défibrillateur, déplacement du patient...

Or, toutes ces interruptions, à la fois courtes et multiples, sont difficiles à quantifier par les soignants, alors qu'elles sont synonymes d'une perte de chance de survie et donc importantes à connaître.

En effet, on comprend aisément qu'un secouriste intervenant sur une personne en arrêt cardiaque n'a pas la possibilité de tenir une comptabilité précise de toutes les durées pendant lesquelles des compressions thoraciques n'ont pas eu lieu, puisqu'il est occupé à sauver la vie de cette personne en lui administrant des compressions thoraciques.

Le problème qui se pose est dès lors de pouvoir connaître le temps total (Ttot) sans compression thoracique (CT), c'est-à-dire la durée totale « no-CT », d'une personne, i.e. un patient, en arrêt cardiaque et ventilée par un appareil d'assistance respiratoire pendant une RCP.

La solution de l'invention concerne alors un appareil d'assistance respiratoire, c'est-à-dire un ventilateur médical, apte à délivrer un flux de gaz à un patient comprenant :

- un conduit d'acheminement de gaz pour acheminer un flux de gaz, en particulier d'air,
- des moyens de mesure conçus pour mesurer au moins un paramètre représentatif du flux de gaz et fournir au moins un signal correspondant audit moins un paramètre représentatif dudit flux de gaz,
- des moyens de traitement de signal conçus pour traiter ledit au moins un signal provenant des moyens de mesure et déduire dudit signal au moins une information relative à une réalisation et/ou à un arrêt de réalisation de contractions thoraciques (CT),
- des moyens de comptage configurés pour comptabiliser des durées d'arrêt ou d'absence de contractions thoraciques (tNCT) pendant lesquelles au moins une information relative à un arrêt ou une absence de contractions thoraciques (CT) est déterminée par les moyens de traitement de signal, et
- des moyens de mémorisation conçus pour enregistrer lesdites durées d'arrêt ou d'absence de contractions thoraciques (tNCT) comptabilisées par les moyens de comptage,

  - et dans lequel les moyens de comptage sont configurés pour déterminer la durée totale (Ttot) d'absence de compressions thoraciques en additionnant les durées d'arrêt ou d'absence de contractions thoraciques (tNCT) pendant lesquelles un arrêt ou une absence de contractions est déterminée.

Les moyens de comptage sont configurés pour comptabiliser plusieurs durées successives S(tNCT) d'arrêt ou d'absence de contractions thoraciques (tNCT) pendant lesquelles un arrêt ou une absence de contractions est déterminée. Dit autrement, les moyens de comptage calculent la somme S(tNCT) des durées successives d'arrêt ou une absence de contractions thoraciques (tNCT), ce qui permet d'obtenir une durée totale (Ttot) d'absence de compressions thoraciques.

Les moyens de comptage sont configurés pour déterminer la durée totale (Ttot) d'absence de compressions thoraciques en additionnant S(tNCT) les durées d'arrêt ou d'absence de contractions thoraciques (tNCT) pendant lesquelles un arrêt ou une absence de contractions est déterminée, c'est-à-dire qu'on calcule la somme S(tNCT) des différents temps sans contraction (tNCT).

Selon le cas, l'appareil d'assistance respiratoire ou ventilateur médical de l'invention peut comprendre l'une ou plusieurs des caractéristiques techniques suivantes :

- les moyens de mesure conçus pour mesurer au moins un paramètre représentatif du flux de gaz choisi parmi la pression du gaz (P) et le débit de gaz (Q), en particulier un débit de gaz insufflé au patient ou un débit de gaz expiré par le patient.

- les moyens de mesure comprennent au moins un capteur de pression de gaz ou un capteur de débit de gaz.

- les moyens de mesure et les moyens de comptage sont configurés pour opérer en continu de manière à détecter et comptabiliser, de manière continue, tout arrêt ou absence de contractions thoraciques.

- il comprend des moyens d'affichage conçus pour afficher au moins une durée d'arrêt ou une absence de contractions thoraciques (tNCT) ou la durée totale (Ttot) d'arrêt ou une absence de contractions thoraciques.

- les moyens de traitement de signal comprennent au moins une carte électronique, de préférence une carte électronique comprenant au moins un microprocesseur mettant en oeuvre au moins un algorithme.

- une (ou la) carte électronique comprend les moyens de comptage.

- les moyens de comptage, encore appelés « dispositif de comptage » ou « compteur », comprennent un dispositif chronomètre.

- les moyens d'affichage comprennent une interface homme/machine ou « IHM ».

- les moyens d'affichage comprennent un écran de visualisation d'informations, de préférence un écran tactile et/ou couleur.

- les moyens de comptage sont configurés pour incrémenter et/ou arrêter un compteur permettant de mesurer le temps total (Ttot) sans contraction thoracique à partir de toutes les durées sans contraction (tNCT) déterminées, pendant un massage cardiaque.

- les moyens de comptage sont configurés pour arrêter d'incrémenter un compteur après arrêt définitif du massage cardiaque sur un patient.

- il comprend des moyens de réglage ou de sélection conçus pour permettre à l'utilisateur, c'est-à-dire au secouriste ou analogue, d'entrer ou de sélectionner des données.

- les moyens de réglage ou de sélection sont conçus pour permettre de renseigner une durée de « no-flow » initial (ou tNF$_{init}$) correspondant au temps (i.e. la durée) écoulé entre la survenue de l'arrêt cardiaque chez le patient et les premières compressions thoraciques réalisées sur ledit patient, par exemple par un secouriste ou une personne témoin présente sur les lieux. La durée de « no-flow » initial (ou tNF$_{init}$) peut être un temps approximatif.

- les moyens de réglage ou de sélection sont conçus pour interagir avec, c'est-à-dire de commander, le ventilateur et pour modifier les paramètres de la ventilation délivrée ou d'y entrer ou sélectionner des données.

- les moyens de réglage ou de sélection sont conçus indiquer au ventilateur un début de massage cardiaque.

- les moyens de comptage sont configurés pour commencer à incrémenter le compteur permettant de mesurer le temps total (Ttot) sans contraction thoracique à partir de toutes les durées sans contraction (tNCT) déterminées, pendant un massage cardiaque, en réponse à une indication par l'utilisateur d'un début de massage cardiaque par activation des moyens de réglage ou de sélection.

- l'interface homme/machine comprend les moyens de réglage ou de sélection, par exemple une ou des touches d'activation ou de sélection, un ou des boutons rotatifs, un ou des curseurs translatifs ou autres.

- l'interface homme/machine comprend un écran tactile comprenant les moyens d'entrée durée, en particulier des touches tactiles.

- les moyens de comptage sont configurés pour additionner la durée de « no-flow » initial (tNF$_{init}$) au temps total (Ttot) sans contraction thoracique pendant le massage cardiaque et en déduire la durée de « no flow » total (tNF$_{tot}$) chez le patient.

- il comprend des moyens de mémorisation conçus pour stocker des informations et/ou des données.

- les moyens de mémorisation comprennent au moins une mémoire de stockage de données, en particulier une mémoire flash ou analogue.

- les moyens de mémorisation configurés pour mémoriser un ou plusieurs modes ventilatoires comme un ou des modes de ventilation volumétriques (VAC), barométriques (VPC, VSAI, CPAP, Duo-Levels..) et/ou intermittents (VACI, PVACI).

- les moyens de traitement de signal sont configurés pour opérer en continu et assurer une détection de contractions thoraciques correspondant à une information de réalisation ou de une non-réalisation de massage cardiaque, et à fournir ladite information aux moyens de calcul de manière à incrémenter ou pas le compteur en fonction de la présence ou de l'absence de contractions thoraciques, c'est-à-dire d'un massage thoracique.

- les moyens de traitement de signal et/ou les moyens de calcul comprennent au moins une carte électronique, de préférence un (micro)contrôleur mettant en oeuvre un ou plusieurs algorithmes.

- l'interface homme-machine (IHM) est apte à et conçue pour afficher des informations incluant au moins une information relative à la durée sans massage cardiaque comprenant la durée de « no-flow » initial (tNF$_{init}$), le temps total (Ttot) sans contraction thoracique pendant le massage cardiaque et/ou la durée de « no flow » total (tNF$_{tot}$) chez le patient.

- il comprend des moyens de réinitialisation configurés pour initialiser ou réinitialiser le compteur, c'est-à-dire le mettre ou remettre à 000 (i.e. zéro).
- les moyens de réinitialisation sont configurés pour initialiser ou réinitialiser le compteur après action de l'utilisateur sur les moyens de réglage ou de sélection pour indiquer au ventilateur un début de massage cardiaque. En d'autres termes, le compteur de durée « tNCT » est initialisé à 000, lors du démarrage du ventilateur, c'est-à-dire lors d'une action de l'utilisateur indiquant un changement de patient.
- il comprend des moyens d'édition de rapport conçus pour éditer, après l'intervention de massage cardiaque, un rapport d'intervention, c'est-à-dire un compte-rendu ou analogue, incluant une ou plusieurs informations de durée choisies parmi la durée de réalisation du massage cardiaque, la durée totale (Ttot) d'absence de compressions thoraciques, la durée de « no-flow » initial ($tNF_{init}$), et/ou la durée de « no flow » total ($tNF_{tot}$).

L'invention va maintenant être mieux comprise grâce à la description détaillée suivante, faite à titre illustratif mais non limitatif, en référence aux figures annexées parmi lesquelles :

- la Figure 1 représente un mode de réalisation d'un appareil d'assistance respiratoire selon la présente invention, dans lequel la source de gaz est une micro-soufflante, et
- la Figure 2 est une représentation graphique de la pression et du débit du flux de gaz insufflé à un patient montrant les périodes avec et sans compressions thoraciques, et des opérations effectuées par le ventilateur de l'invention.

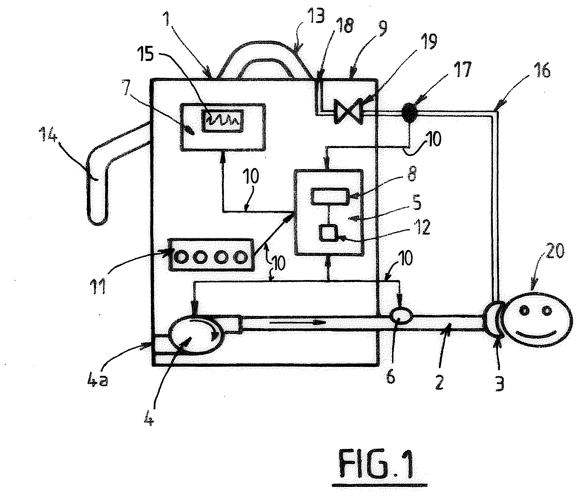

La Figure 1 schématise un mode de réalisation d'un appareil d'assistance ventilatoire ou ventilateur médical 1 selon la présente invention.

Il comprend une source de gaz 4 qui est ici une micro-soufflante motorisée, encore appelée turbine, délivrant un flux de gaz d'assistance respiratoire, typiquement un flux d'air ou d'air enrichi en oxygène. L'air est aspiré par la micro-soufflante via un (ou des) orifice d'entrée 4a aménagé dans la carcasse 9 du ventilateur 1.

De façon alternative (non montrée), la source de gaz 4 comprendre une vanne pilotée alimentée en gaz, via un conduit interne, lui-même en communication fluidique avec un réservoir de gaz ou une prise murale de fourniture de gaz reliée à un réseau de canalisations de gaz, par l'intermédiaire d'un conduit souple raccordant le réservoir de gaz ou la prise murale au conduit interne.

Le circuit ventilatoire 2, 16, encore appelé circuit patient, comprenant un ou plusieurs passages, conduits ou lignes de gaz, permet de relier fluidiquement la source de gaz 4 du ventilateur 1 aux voies aériennes d'un patient 20, par l'intermédiaire d'une interface patient 3, par exemple un masque respiratoire ou une sonde d'intubation.

Le circuit ventilatoire 2, 16 comprend au moins une branche inspiratoire 2 permettant de convoyer le gaz respiratoire au patient 20. Il peut comporter également une branche expiratoire 16 conçue pour recueillir les gaz expirés par le patient 20 qui sont riches en $CO_2$.

La branche expiratoire 16 comprend un capteur de débit expiratoire 17, par exemple un capteur à fil chaud, relié électriquement aux moyens de pilotage 5, telle une carte électronique, ainsi qu'une valve expiratoire 19 pilotée par les moyens de pilotage 5. La branche expiratoire 16 communique, à son extrémité aval, avec l'atmosphère via un orifice de sortie de gaz 18, alors que son extrémité amont est reliée à la branche inspiratoire 2, via une pièce en Y, soit directement à l'interface patient 3.

Des moyens de mesure 6 sont prévus, tel un capteur de pression et/ou de débit, lesquels sont aptes à et conçus pour mesurer au moins un paramètre représentatif du flux de gaz, en particulier la pression du gaz ou le débit de gaz, que ce soit le débit insufflé par le respirateur et/ou le débit de gaz expiré par le patient 20, et à délivrer au moins un signal représentatif dudit au moins un paramètre mesuré.

La mesure se fait dans la branche inspiratoire 2 du circuit de gaz ventilatoire 2, 16 de manière à permettre une mesure de la pression ou du débit gazeux dans ladite branche inspiratoire 2 faisant office de conduit d'acheminement de gaz. Dans le mode de réalisation illustré sur la Figure 1, les moyens de mesure 6 sont agencés hors du ventilateur ; toutefois, ils peuvent également se trouver dans le ventilateur 1, selon un autre mode de réalisation.

Une fois la mesure du (ou des) paramètre(s) représentatif(s) du flux de gaz opérée, ce paramètre mesuré est converti en au moins un signal représentatif du flux de gaz, qui est alors transmis à et analysé par des moyens de traitement de signal 8, c'est-à-dire typiquement la carte électronique faisant office de moyens de pilotage 5, pour en déduire au moins une information relative à un massage cardiaque opéré sur un patient en arrêt cardiaque, en particulier au moins une information relative à une réalisation et/ou à un arrêt de réalisation de contractions thoraciques CT.

Les moyens de traitement de signal 8 font donc partie des moyens de pilotage 5 du ventilateur 1 et comprennent une ou plusieurs cartes électroniques à microprocesseur(s) mettant en oeuvre un ou des algorithmes.

Le ventilateur 1 comprend en outre des moyens de comptage, comme par exemple un compteur ou un chronomètre, qui coopèrent avec les moyens de traitement de signal 8.

Plus précisément, les moyens de comptage sont configurés pour, c'est-à-dire conçus pour et apte à, comptabiliser chaque durée d'arrêt ou d'absence de contractions thoraciques (tNCT) en réponse à la détection d'une information relative à un arrêt ou une absence de contractions thoraciques (CT) par les moyens de traitement de signal 8.

Toutes les durées successives tNCT d'arrêt ou d'absence de contractions thoraciques sont comptabilisées et additionnées pour obtenir une durée totale Ttot d'absence de compressions thoraciques.

Des moyens de mémorisation 12, telle une mémoire flash, permettent d'enregistrer la ou lesdites durées d'arrêt ou d'absence de contractions thoraciques (tNCT) comptabilisée par les moyens de comptage, ainsi que la durée totale Ttot d'absence de compressions thoraciques.

Le ventilateur 1 comprend en outre une interface homme/machine comprenant une ou des touches 11 de commande, activables par l'utilisateur, c'est-à-dire le personnel soignant, comme un secouriste par exemple, ainsi qu'un écran de visualisation d'informations 7, de préférence l'interface homme/machine comprend un écran tactile à affichage en couleurs dont certaines zones constituent des touches de commande tactiles.

Lorsque les moyens de traitement de signal 8 ont traité le (ou les) signal provenant des moyens de mesure 6 et en ont déduit une information caractéristique d'un massage cardiaque opéré sur le patient 20, les moyens de comptage commencent alors à comptabiliser les différentes tNCT durées d'arrêt ou d'absence de contractions thoraciques, pendant lesquelles un arrêt ou une absence de contractions est déterminée, c'est-à-dire à les additionner les unes aux autres de manière à pouvoir en déduire une durée totale Ttot d'absence de compressions thoraciques.

De préférence, les moyens de mesure 6 et les moyens de comptage sont configurés pour opérer en continu de manière à détecter de manière continue tout arrêt ou absence de contractions thoraciques, ainsi que toute reprise de massage cardiaque.

De préférence, les moyens de comptage sont configurés pour commencer à incrémenter le compteur permettant de mesurer le temps total Ttot sans contraction thoracique à partir de toutes les durées sans contraction tNCT déterminées, pendant un massage cardiaque, en réponse à une indication par l'utilisateur d'un lancement de mode ventilatoire spécifique à une réanimation cardio pulmonaire, par activation de moyens de réglage ou de sélection, par exemple après que l'utilisateur ait appuyé sur une touche indiquant un début de massage cardiaque.

Enfin, le ventilateur 1 affiche sur l'interface homme/machine, une durée tNCT d'arrêt ou d'absence de contractions thoraciques ou la durée totale (Ttot) d'arrêt ou d'absence de contractions thoraciques correspondant à la somme S(tNCT) de toutes les durée tNCT d'arrêt ou d'absence de contractions thoraciques.

Des moyens d'édition de rapport, tels qu'un affichage du rapport en fin d'intervention ou la possibilité d'extraire un rapport de fin d'intervention sur un dispositif de stockage USB ou analogue sont prévus. Ils sont préférentiellement conçus pour éditer, après l'intervention de massage cardiaque, un rapport d'intervention, c'est-à-dire un compte-rendu ou analogue, incluant une ou plusieurs informations de durée choisies parmi la durée de réalisation du massage cardiaque, la durée totale Ttot d'absence de compressions thoraciques, la durée $tNF_{init}$ de « no-flow » initial, et/ou la durée $tNF_{tot}$ de « no flow » total.

La transmission des signaux entre les différents composants du ventilateur 1 se fait via des connectiques adaptées, c'est-à-dire des les liaisons électriques 10, telles que câbles ou autres.

Le ventilateur 1 peut comprendre également une poignée de portage 13 et/ou un système d'accrochage 14, tel un crochet ou analogue, servant à accrocher l'appareil à un support. Le transport du ventilateur 1 peut se faire aussi au sein d'une mallette rigide ou d'une sacoche souple.

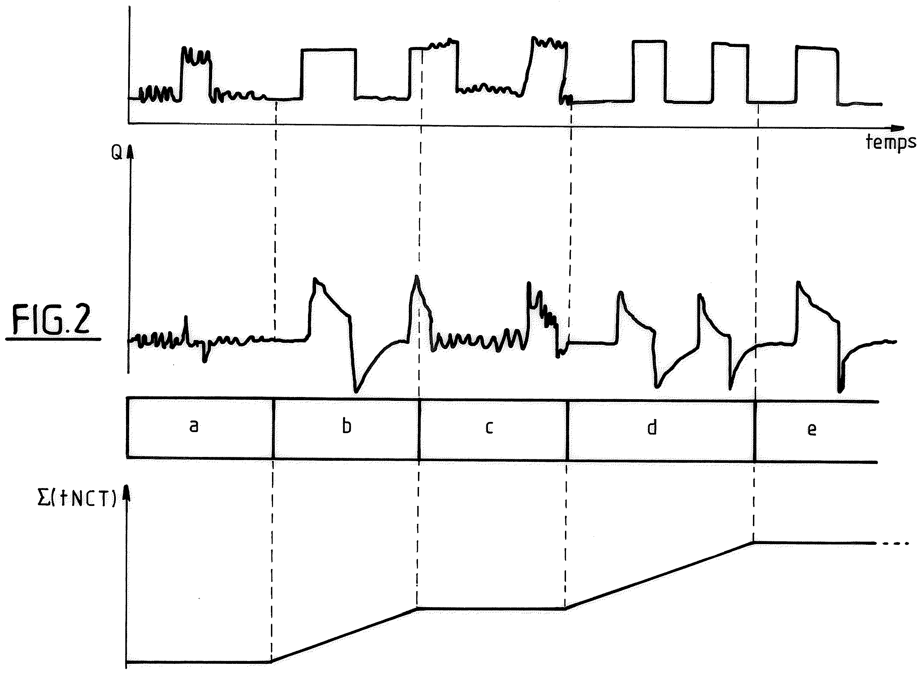

La Figure 2 est une représentation graphique de la pression P et du débit Q du flux de gaz insufflé à un patient au fil du temps t, au moyen d'un ventilateur selon l'invention, par exemple le ventilateur de la Figure 1, qui permet de distinguer les périodes avec (phases a et c) et sans (phase b) compressions thoraciques et une représentation des opérations effectuées par le respirateur.

Plus précisément, lorsque le respirateur 1 détecte la présence d'un massage cardiaque (phases a et c) en analysant le signal de débit Q et/ou de pression P provenant du capteur de débit ou de pression utilisé 6, la durée tNCT passée sans compressions thoraciques n'est pas incrémentée.

A l'inverse, lorsque le respirateur ne détecte pas de massage cardiaque (phase b) en analysant le signal de débit Q et/ou de pression P, la durée tNCT passée sans compression thoracique est incrémentée, c'est-à-dire qu'elle s'additionne aux autres durées tNCT qui ont pu être précédemment relevées et additionnées les unes aux autres de manière à déterminer la durée totale Ttot reflétant l'absence de compressions thoraciques CT.

En cas de période prolongée (1 minute par exemple) sans compressions thoraciques (phase d), le ventilateur considère que le patient a été réanimé ou que la tentative de réanimation a été abandonnée. Dans cette phase e , le compteur tNCT n'est plus incrémenté.

La connaissance de tNCT, permettra de calculer et d'afficher le pourcentage du temps de réanimation pendant lequel des compressions sont réalisées de manière à encourager les soignants à ne jamais interrompre les compressions thoraciques pour se rapprocher de la valeur idéal de 100%. Cet indice que nous appellerons %CT est calculé de la manière suivante :

$$\%CT = \frac{tNCT}{Ttot} * 100$$

Ensuite, la durée de réalisation du massage cardiaque, la durée totale Ttot d'absence de compressions thoraciques, la durée $tNF_{init}$ de « no-flow » initial, et/ou la durée tNF$_{tot}$ de « no flow » total peuvent être affichées sur l'écran tactile 7 du ventilateur 1 et/ou faire l'objet d'un rapport d'intervention destiné au personnel soignant.

D'une façon générale, l'appareil de ventilation artificielle de l'invention sont parfaitement adaptés à une utilisation lors d'un massage cardiaque sur un patient ventilé, pour assurer un monitorage, c'est-à-dire un suivi, du massage cardiaque pratiqué par les secouristes, tel que médecin du SAMU, pompier, infirmier ou autre.

**Revendications**

1. Appareil d'assistance respiratoire (1) apte à délivrer un flux de gaz à un patient (P) comprenant :

    - un conduit d'acheminement de gaz (2) pour acheminer un flux de gaz,
    - des moyens de mesure (6) conçus pour mesurer au moins un paramètre représentatif du flux de gaz et fournir au moins un signal correspondant audit moins un paramètre représentatif dudit flux de gaz, et
    - des moyens de traitement de signal (8) conçus pour traiter ledit au moins un signal provenant des moyens de mesure (6) et déduire dudit signal au moins une information relative à une réalisation et/ou à un arrêt de réalisation de contractions thoraciques (CT),

    **caractérisé en ce qu'**il comprend en outre :

    - des moyens de comptage configurés pour comptabiliser des durées d'arrêt ou d'absence de contractions thoraciques (tNCT) pendant lesquelles au moins une information relative à un arrêt ou une absence de contractions thoraciques (CT) est déterminée par les moyens de traitement de signal (8), et
    - des moyens de mémorisation (12) conçus pour enregistrer lesdites durées d'arrêt ou d'absence de contractions thoraciques (tNCT) comptabilisées par les moyens de comptage,

    et dans lequel les moyens de comptage sont configurés pour déterminer la durée totale (Ttot) d'absence de compressions thoraciques en additionnant les durées d'arrêt ou d'absence de contractions thoraciques (tNCT) pendant lesquelles un arrêt ou une absence de contractions est déterminée.

2. Appareil selon la revendication précédente, **caractérisé en ce que** les moyens de mesure (6) conçus pour mesurer au moins un paramètre représentatif du flux de gaz choisi parmi la pression du gaz (P) et le débit de gaz (Q), de préférence les moyens de mesure (6) comprennent au moins un capteur de pression de gaz ou un capteur de débit de gaz.

3. Appareil selon l'une des revendications précédentes, **caractérisé en ce que** les moyens de mesure (6) et les moyens de comptage sont configurés pour opérer en continu de manière à détecter et comptabiliser, de manière continue, tout arrêt ou absence de contractions thoraciques.

4. Appareil selon l'une des revendications précédentes, **caractérisé en ce qu'**il comprend des moyens d'affichage (7) conçus pour afficher au moins une durée d'arrêt ou une absence de contractions thoraciques (tNCT) ou la durée totale (Ttot) d'arrêt ou d'absence de contractions thoraciques.

5. Appareil selon l'une des revendications précédentes, **caractérisé en ce que** les moyens de traitement de signal (8) comprennent au moins une carte électronique, de préférence une carte électronique comprenant au moins un microprocesseur mettant en oeuvre au moins un algorithme.

6. Appareil selon la revendication 4, **caractérisé en ce que** les moyens d'affichage (7) comprennent un écran de visualisation d'informations, de préférence un écran tactile et/ou couleur.

7. Appareil selon l'une des revendications précédentes, **caractérisé en ce que** les moyens de comptage sont configurés pour commencer à incrémenter le compteur permettant de mesurer le temps total (Ttot) sans contraction thoracique à partir de toutes les durées sans contraction (tNCT) déterminées, pendant un massage cardiaque, en réponse à une indication par l'utilisateur d'un début de massage cardiaque par activation de moyens de réglage ou de sélection.

8. Appareil selon l'une des revendications précédentes, **caractérisé en ce que** les moyens de comptage comprennent un dispositif compteur ou un dispositif chronomètre.

9. Appareil selon la revendication 5, **caractérisé en ce que** la carte électronique comprend les moyens de comptage.

**Patentansprüche**

1. Vorrichtung zur Atmungsunterstützung (1), die zum Zuführen einer Gasströmung zu einem Patienten (P) imstande ist, umfassend:

    - eine Gasförderleitung (2) zum Fördern einer Gasströmung,
    - Messmittel (6), die zum Messen mindestens eines für die Gasströmung repräsentativen Parameters und zum Liefern mindestens eines Signals, das dem mindestens einen für die Gasströmung repräsentativen Parameter entspricht, konzipiert sind, und

- Signalverarbeitungsmittel (8), die zum Verarbeiten des mindestens einen von den Messmitteln (6) stammenden Signals und zum Ableiten mindestens einer Information bezüglich einer Ausführung und/oder einem Stillstand der Ausführung von Thoraxkontraktionen (CT) aus dem Signal konzipiert sind,

**dadurch gekennzeichnet, dass** sie darüber hinaus umfasst:

- Zählmittel, die dazu eingerichtet sind, die Zeiträume eines Stillstands oder eines Fehlens von Thoraxkontraktionen (tNCT) zu zählen, in denen mindestens eine Information bezüglich eines Stillstands oder eines Fehlens von Thoraxkontraktionen (CT) durch die Signalverarbeitungsmittel (8) bestimmt wird, und

- Speichermittel (12), die zum Speichern der von den Zählmitteln gezählten Zeiträume eines Stillstands oder eines Fehlens von Thoraxkontraktionen (tNCT) konzipiert sind,

und wobei die Zählmittel dazu eingerichtet sind, die Gesamtdauer (Ttot) des Fehlens von Thoraxkontraktionen zu bestimmen, indem sie die Zeiträume eines Stillstands oder eines Fehlens von Thoraxkontraktionen (tNCT), in denen ein Stillstand oder ein Fehlen von Kontraktionen bestimmt wird, addieren.

2. Vorrichtung nach dem vorstehenden Anspruch, **dadurch gekennzeichnet, dass** die Messmittel (6), die zum Messen mindestens eines für die Gasströmung repräsentativen Parameters konzipiert sind, der aus dem Gasdruck (P) und dem Gasdurchsatz (Q) ausgewählt ist, wobei die Messmittel (6) vorzugsweise mindestens einen Gasdrucksensor oder einen Gasdurchsatzsensor umfassen.

3. Vorrichtung nach einem der vorstehenden Ansprüche, **dadurch gekennzeichnet, dass** die Messmittel (6) und die Zählmittel für den kontinuierlichen Betrieb eingerichtet sind, um jeden Stillstand oder jedes Fehlen von Thoraxkontraktionen kontinuierlich zu erfassen und zu zählen.

4. Vorrichtung nach einem der vorstehenden Ansprüche, **dadurch gekennzeichnet, dass** sie Anzeigemittel (7) umfasst, die zum Anzeigen einer Dauer eines Stillstands oder eines Fehlens von Thoraxkontraktionen (tNCT) oder der Gesamtdauer (Ttot) eines Stillstands oder eines Fehlens von Thoraxkontraktionen konzipiert sind.

5. Vorrichtung nach einem der vorstehenden Ansprüche, **dadurch gekennzeichnet, dass** die Signalverarbeitungsmittel (8) mindestens eine elektronische Karte, vorzugsweise eine elektronische Karte umfassen, die mindestens einen Mikroprozessor umfasst, der mindesten einen Algorithmus ausführt.

6. Vorrichtung nach Anspruch 4, **dadurch gekennzeichnet, dass** die Anzeigemittel (7) einen Bildschirm zur Visualisierung von Informationen, vorzugsweise einen berührungsempfindlichen und/oder einen Farbbildschirm, umfassen.

7. Vorrichtung nach einem der vorstehenden Ansprüche, **dadurch gekennzeichnet, dass** die Zählmittel dazu eingerichtet sind, mit dem Erhöhen des Zählers, wodurch das Messen der Gesamtzeit (Ttot) ohne Thoraxkontraktion ausgehend von allen bestimmten Zeiträumen ohne Kontraktion (tNCT) ermöglicht wird, während einer Herzdruckmassage als Reaktion auf eine Angabe durch den Benutzer eines Beginns der Herzdruckmassage durch eine Aktivierung von Regel- oder Auswahlmitteln zu beginnen.

8. Vorrichtung nach einem der vorstehenden Ansprüche, **dadurch gekennzeichnet, dass** die Zählmittel eine Zähleinrichtung oder eine Zeitmesseinrichtung umfassen.

9. Vorrichtung nach Anspruch 5, **dadurch gekennzeichnet, dass** die elektronische Karte die Zählmittel umfasst.

## Claims

1. Respiratory assistance apparatus (1) capable of delivering a stream of gas to a patient (P) comprising:

- a gas delivery conduit (2) for conveying a stream of gas,
- measuring means (6) designed to measure at least one parameter representative of the stream of gas and provide at least one signal corresponding to said at least one parameter representative of said stream of gas, and
- signal processing means (8) designed to process said at least one signal coming from the measuring means (6) and to deduce from said signal at least one item of information relative to performance and/or to discontinued performance of chest contractions (CT),

**characterized in that** it further comprises:

- calculating means configured to calculate durations of the discontinuation or absence of chest contractions (tNCT) during which at least one item of information relative to a discontinuous or an absence of chest contractions (CT) is determined by the signal processing means (8), and

- means for memorising (12) designed to record said durations of discontinuation or of absence of chest contractions (tNCT) counted by the calculating means,

and wherein the calculating means are configured to determine the total duration (Ttot) of absence of chest compressions by adding the durations of discontinuation or of absence of chest contractions (tNCT) during which a discontinuation or an absence of contractions is determined.

2. Apparatus according to the preceding claim, **characterized in that** the measuring means (6) designed to measure at least one parameter representative of the stream of gas chosen from the pressure of the gas (P) and the stream rate of the gas (Q), preferably the measuring means (6) comprise at least one gas pressure sensor or a gas stream rate sensor.

3. Apparatus according to one of the preceding claims, **characterized in that** the measuring means (6) and the calculating means are configured to operate continuously so as to detect and count, continuously, any discontinuation or absence of chest contractions.

4. Apparatus according to one of the preceding claims, **characterized in that** it comprises display means (7) designed to display at least one duration of discontinuation or an absence of chest contractions (tNCT) or the total duration (Ttot) of discontinuation or absence of chest contractions.

5. Apparatus according to one of the preceding claims, **characterized in that** the signal processing means (8) comprise at least one electronic board, preferably an electronic board comprising at least one microprocessor implementing at least one algorithm.

6. Apparatus according to claim 4, **characterized in that** the display means (7) comprise a screen for viewing information, preferably a touch and/or colour screen.

7. Apparatus according to one of the preceding claims, **characterized in that** the calculating means are configured to start incrementing the counter making it possible to measure the total time (Ttot) without chest contraction from all of the determined durations without contraction (tNCT), during a cardiac massage, in response to an indication by the user of the beginning of a cardiac massage by activation of the adjustment or selection means.

8. Apparatus according to one of the preceding claims, **characterized in that** the calculating means comprise a counting device or a timing device.

9. Apparatus according to claim 5, **characterized in that** the electronic board comprises the calculating means.

FIG.1

EP 3 218 036 B1

FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3000893 A1
- WO 2013025406 A1
- US 2013310718 A1
- EP 2198823 A1